# FASCICULE DE BREVET EUROPEEN

(11) **EP 2 697 642 B1**
(45) Date de publication et mention de la délivrance du brevet: **04.11.2015**
(21) Numéro de dépôt: 12718298.8
(22) Date de dépôt: 04.04.2012
(51) Int. Cl.: G01N 33/00, G01W 1/02

(54) **PROCÉDÉ DE MESURE D'HUMIDITÉ RELATIVE ET DE TEMPÉRATURE DE L'AIR DANS UN MILIEU DIPHASIQUE**
VERFAHREN ZUR MESSUNG DER RELATIVEN FEUCHTIGKEIT UND TEMPERATUR DER LUFT IN EINEM ZWEIPHASIGEN MEDIUM
METHOD FOR MEASURING RELATIVE HUMIDITY AND TEMPERATURE OF THE AIR IN A DIPHASIC MEDIUM

(30) Priorité: 12.04.2011 FR 1153170
(43) Date de publication de la demande: 19.02.2014
(73) Titulaire: Institut National de Recherche en Sciences et Technologies pour l'Environnement et l'Agriculture, 92761 Antony Cedex (FR)
(72) Inventeur: GAHARTIAN, Jérôme, F-75003 Paris (FR); TISSOT, Julien, F-54740 Lemainville (FR)
(74) Mandataire: Laget, Jean-Loup
(86) Numéro de dépôt international: PCT/FR2012/050726
(87) Numéro de publication internationale: WO 2012/140348

(56) Documents cités:
- WO-A1-00/36439
- JP-A- 2006 105 745
- US-A- 3 465 591
- US-A- 5 148 710
- US-B1- 6 247 360

## Description

L'invention concerne un procédé de mesure d'humidité relative et de température de l'air en milieu diphasique, notamment en milieu air/eau et plus particulièrement en milieu air/gouttelettes d'eau.

Dans un tel milieu, par exemple dans le cas de la brumisation d'eau dans l'air, la température de l'eau et celle de l'air sont différentes tant que l'air n'a pas atteint la saturation en vapeur d'eau. Lorsque les températures de l'air et de l'eau sont différentes, un capteur de température indique normalement la température de l'air. Cependant, s'il est impacté par une gouttelette d'eau, le capteur indique la température de la gouttelette d'eau et non celle de l'air. De la même manière, un capteur d'humidité relative indique normalement l'humidité relative de l'air. S'il est impacté par une gouttelette d'eau, le capteur indique une humidité relative de 100 % correspondant à celle de l'eau.

Un but de l'invention est de proposer un procédé de mesure de l'humidité relative et de la température dans un milieu diphasique air/eau, qui soit affranchi du risque de mesurer l'humidité relative et la température de la phase aqueuse du milieu.

L'invention a pour objet un procédé de mesure de l'humidité relative et de la température de l'air dans un milieu diphasique d'air et de gouttelettes d'eau, au moyen d'un capteur d'humidité relative et d'un capteur de température disposés à l'intérieur d'un tube vertical, caractérisé en ce que le milieu diphasique est soumis à un mouvement ascensionnel dans le tube, à une vitesse inférieure à la vitesse d'équilibre des gouttelettes d'eau dans un air ascensionnel.

De manière avantageuse, le mouvement ascensionnel est assuré par aspiration à la partie supérieure du tube.

D'autres buts, détails, caractéristiques et avantages de l'invention ressortent de la description suivante d'un mode de réalisation particulier de l'invention, donné à titre illustratif et non limitatif, en référence aux dessins annexés.

Sur les dessins :
- la figure 1 est un schéma simplifié d'un dispositif de mesure d'humidité relative et de température de l'air dans un milieu diphasique air/eau ;
- la figure 2 est un diagramme représentatif de la vitesse d'équilibre des gouttelettes d'eau, dans un air ascensionnel, en fonction de leur diamètre.

Le dispositif de mesure d'humidité relative et de température de l'air dans un milieu diphasique comporte un capteur d'humidité relative 1, et un capteur de température 2, reliés à un mesureur 3. Selon l'invention, en milieu diphasique air/eau, le capteur d'humidité relative 1 et le capteur de température 2 sont placés à l'intérieur d'un tube 4 sensiblement vertical et soumis à une aspiration à sa partie supérieure pour provoquer une ascension d'air dans le tube 4. Cette ascension d'air dans le tube est assurée par une pompe, non représentée, à une vitesse inférieure à la vitesse d'équilibre des gouttelettes d'eau dans un air ascensionnel, pour éviter une remontée des gouttelettes d'eau dans le tube 4. Il en résulte que seul l'air circule dans le tube. Une première conséquence est que les mesures de la température et de l'humidité relative ne sont pas perturbées par l'impact de gouttelettes d'eau sur les capteurs. Une deuxième conséquence est que le renouvellement de l'air dans le tube permet de mesurer la valeur moyenne de la température et de l'humidité relative de l'air circulant en dehors du tube 4. L'ensemble du tube 4 et de la pompe constitue un dispositif de protection des capteurs 1, 2 contre l'impact des gouttelettes d'eau en suspension dans l'air.

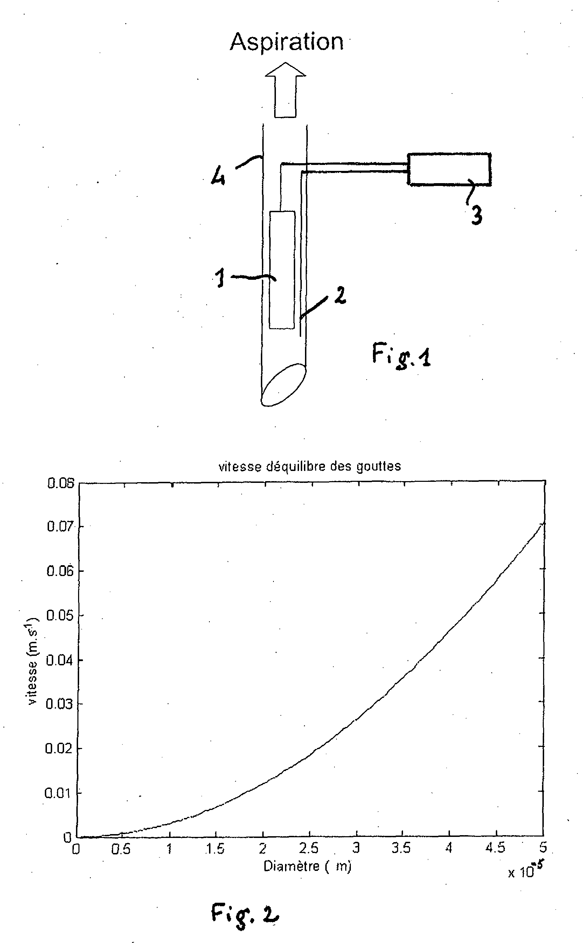

Sur la figure 2, la courbe représente la vitesse d'équilibre des gouttelettes d'eau dans un air ascensionnel, en fonction du diamètre des gouttelettes d'eau. Pour des gouttelettes d'eau d'un diamètre donné, la vitesse de l'air dans le tube doit être inférieure à la vitesse d'équilibre : sur la figure 2, le point représentatif de la vitesse de l'air dans le tube doit être en dessous de la courbe.

Dans un mode de réalisation donné à titre d'exemple non limitatif, le tube 4 a un diamètre de l'ordre de 1 cm, et une longueur d'environ 10 cm. La distance moyenne entre l'ouverture inférieure du tube 4 et les capteurs 1, d'humidité relative, et 2, de température, est d'environ deux fois le diamètre du tube 4) c'est-à-dire d'environ 2 cm. Pour un diamètre moyen des gouttelettes d'eau de 3.10⁻⁵ m, la vitesse de circulation de l'air dans le tube 4 est de l'ordre de 0,02 m.s⁻¹.

Selon l'invention, le tube 4 est traversé par un courant d'air ascensionnel, de vitesse inférieure à la vitesse d'équilibre des gouttelettes d'eau dans un air ascensionnel. Le courant d'air est assuré par aspiration au moyen d'une pompe, à la partie supérieure du tube 4.

## Revendications

1. Procédé de mesure de l'humidité relative et de la température de l'air dans un milieu diphasique d'air et de gouttelettes d'eau, au moyen d'un capteur d'humidité relative et d'un capteur de température disposés à l'intérieur d'un tube vertical, **caractérisé en ce que** le milieu diphasique est soumis à un mouvement ascensionnel dans le tube, à une vitesse inférieure à la vitesse d'équilibre des gouttelettes d'eau dans un air ascensionnel.

2. Procédé selon la revendication 1 **caractérisé en ce que** le mouvement ascensionnel est assuré par aspiration à la partie supérieure du tube.

## Patentansprüche

1. Verfahren zur Messung der relativen Feuchtigkeit und der Temperatur der Luft in einem zweiphasigen Luft- und Wassertröpfchenmedium mit einem Sensor der relativen Feuchtigkeit und einem Sensor der Temperatur, die im Innern eines vertikalen Rohrs angeordnet sind, **dadurch gekennzeichnet, dass** das zweiphasige Medium in dem Rohr einer aufsteigenden Bewegung mit einer Geschwindigkeit, die kleiner ist als die Gleichgewichtsgeschwindigkeit der Wassertröpfchen in einer aufsteigenden Luft, ausgesetzt wird.

2. Verfahren nach Anspruch 1, **dadurch gekennzeichnet, dass** die aufsteigende Bewegung durch Ansaugen im oberen Teil des Rohrs gesichert ist.

## Claims

1. A method for measuring the relative humidity and the temperature of the air in a diphasic medium of air and water droplets, using a relative humidity sensor and a temperature sensor positioned inside a vertical tube, **characterized in that** the diphasic medium is subject to an upward movement in the tube, at a speed lower than the equilibrium speed of the water droplets in rising air.

2. The method according to claim 1, **characterized in that** the upward movement is provided by suction at the upper part of the tube.
